(19) Europäisches Patentamt European Patent Office Office européen des brevets

(11) **EP 4 480 509 A1**

(12) **EUROPEAN PATENT APPLICATION**
published in accordance with Art. 153(4) EPC

(43) Date of publication:
**25.12.2024 Bulletin 2024/52**

(21) Application number: **22935623.3**

(22) Date of filing: **25.10.2022**

(51) International Patent Classification (IPC):
***A61L 27/22*** *(2006.01)*    ***A61L 27/56*** *(2006.01)*
***C07K 14/78*** *(2006.01)*    ***C12N 15/12*** *(2006.01)*

(52) Cooperative Patent Classification (CPC):
***A61L 27/22; A61L 27/56;*** C07K 14/435;
C07K 14/78

(86) International application number:
**PCT/JP2022/039789**

(87) International publication number:
**WO 2023/188492 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.03.2022 JP 2022057374**
          **01.04.2022 JP 2022062058**

(71) Applicant: **FUJIFILM Corporation**
**Tokyo 106-8620 (JP)**

(72) Inventors:
• **ITO, Sayami**
  **Ashigarakami-gun, Kanagawa 258-8577 (JP)**
• **OKAMURA, Ai**
  **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **TISSUE RESTORATION MATERIAL AND TISSUE RESTORATION MATERIAL PRODUCTION METHOD**

(57)    A tissue repair material and a manufacturing method of a tissue repair material, in which a crosslinked gelatin is contained in the tissue repair material and in an elution curve of the crosslinked gelatin, which is obtained by gel permeation chromatography under the following conditions, at least one absorption peak with an absorbance of 0.025 or more is present in a molecular weight range of 14 kDa or more and 51 kDa or less.

FIG. 1

EP 4 480 509 A1

**Description**

**BACKGROUND OF THE INVENTION**

1. Field of the Invention

The present disclosure relates to a tissue repair material and a manufacturing method of a tissue repair material.

2. Description of the Related Art

Currently, a practical application of regenerative medicine for regeneration of biological tissues or organs, which have been dysfunctional or malfunctioning, has progressed. In biological tissues which cannot be recovered by natural healing ability of the living body alone, the regenerative medicine is a novel medical technology that recreates the same form and function as the original tissue using three factors of cells, scaffolds, and growth factors.

Among these, bone regeneration in the orthopedic field or the dental field is a field attracting great attention in the regenerative medicine field. In a case where the bone disease occurs in legs or lower back, it is not possible to walk because of a bone defect caused by the disease, and in a case where bone disease occurs in periodontal tissue, since it is difficult to eat, the bone disease causes a marked deterioration in quality of life (QOL).

In the field of regenerative medicine, collagen or gelatin having high bioaffinity is used. For example, WO2013/137268A discloses a tissue repair material containing crosslinked gelatin.

**SUMMARY OF THE INVENTION**

Meanwhile, the tissue repair material is required to have excellent tissue repair ability, and for example, it is required that a tissue repair rate after elapse of about 4 weeks after the application of the tissue repair material to a site where a tissue is missing or damaged is high (hereinafter, also referred to as tissue repairability).

An object to be achieved by an embodiment of the present disclosure is to provide a tissue repair material having excellent tissue repairability and a manufacturing method of a tissue repair material.

The present disclosure includes the following aspects.

<1> A tissue repair material comprising crosslinked gelatin, in which in an elution curve of the crosslinked gelatin, which is obtained by gel permeation chromatography under the following conditions, at least one absorption peak with an absorbance of 0.025 or more is present in a molecular weight range of 14 kDa or more and 51 kDa or less.

(Conditions)
Column length: 150 mm
Column inner diameter: 4.6 mm
Filler: ethylene bridged (BEH) particles
Particle size of the filler: 1.7 $\mu$m
Pore size of the filler: 200 Å
Column temperature: 30°C
Mobile phase: 0.1 mol/L sodium sulfate, 0.01 mol/L sodium dihydrogen phosphate, and 1% by mass of sodium dodecyl sulfate, pH 5.3
Flow rate: 0.1 mL/minute
Detector: wavelength-tunable UV detector (TUV)
Measuring wavelength: 215 nm
Adjustment of measurement sample: The tissue repair material is filled into a tube having a capacity of 15 mL, 5 mL of water for injection is added to the tube, and the tube is incubated at 37°C for 24 hours. 1 mL of a supernatant is collected by a low-adsorptive tube after mixing with inversion and filtered through a 0.22 $\mu$m filter.
Amount of the measurement sample: 5 $\mu$L

<2> The tissue repair material according to <1>, in which an absorption peak is present in a molecular weight range of 14 kDa or more and less than 28 kDa and an absorption peak is present in a molecular weight range of 28 kDa or more and less than 43 kDa.

<3> The tissue repair material according to <1> or <2>, in which a water absorption rate of the crosslinked gelatin is 450% or more.

<4> The tissue repair material according to any one of <1> to <3>, in which a residual rate of the crosslinked gelatin after a decomposition treatment for 3 hours using 1 mol/liter (L) hydrochloric acid is 60% or less.

<5> The tissue repair material according to any one of <1> to <4>, in which the gelatin is one or more selected from the group consisting of a peptide consisting of an amino acid sequence set forth in SEQ ID NO: 1, a peptide consisting of an amino acid sequence in which one or several amino acids are deleted, substituted, or added from the amino acid sequence set forth in SEQ ID NO: 1, and having bioaffinity, and a peptide consisting of an amino acid sequence having amino acid sequence identity of 80% or more with a partial amino acid sequence consisting of the 4th to 192nd amino acid residues in the amino acid sequence set forth in SEQ ID NO: 1, and having bioaffinity.

<6> The tissue repair material according to any one of <1> to <5>, in which in the elution curve of the crosslinked gelatin, a ratio of an area at a molecular weight of 14 kDa or more and 43 kDa or less to an area at a molecular weight of more than 43 kDa and 51 kDa or less is 2.5 or more.

<7> A manufacturing method of a tissue repair material comprising a step of heating gelatin under conditions of 100°C to 170°C and 2 hours to 24 hours to obtain crosslinked gelatin, and a step of irradiating the crosslinked gelatin with radiation having a dose of 5 kGy to 50 kGy.

<8> The manufacturing method of a tissue repair material according to <7>, in which the radiation is a γ-ray or an electron beam.

<9> The manufacturing method of a tissue repair material according to <7> or <8>, in which, in a molecular weight range of 14 kDa or more and 51 kDa or less in an elution curve of the crosslinked gelatin in a tissue repair material manufactured by the manufacturing method of a tissue repair material according to <7> or <8>, which is obtained by gel permeation chromatography under the following conditions, an absorption peak is present in a molecular weight range of 14 kDa or more and less than 28 kDa and an absorption peak is present in a molecular weight range of 28 kDa or more and less than 43 kDa.

(Conditions)
Column length: 150 mm
Column inner diameter: 4.6 mm
Filler: ethylene bridged (BEH) particles
Particle size of the filler: 1.7 μm
Pore size of the filler: 200 Å
Column temperature: 30°C
Mobile phase: 0.1 mol/L sodium sulfate, 0.01 mol/L sodium dihydrogen phosphate, and 1% by mass of sodium dodecyl sulfate, pH 5.3
Flow rate: 0.1 mL/minute
Detector: wavelength-tunable UV detector (TUV)
Measuring wavelength: 215 nm
Adjustment of measurement sample: The tissue repair material is filled into a tube having a capacity of 15 mL, 5 mL of water for injection is added to the tube, and the tube is incubated at 37°C for 24 hours. 1 mL of a supernatant is collected by a low-adsorptive tube after mixing with inversion and filtered through a 0.22 μm filter.
Amount of the measurement sample: 5 μL

<10> The manufacturing method of a tissue repair material according to any one of <7> to <9>, in which a water absorption rate of the crosslinked gelatin after the irradiation with radiation is 450% or more.

<11> The manufacturing method of a tissue repair material according to any one of <7> to <10>, in which an acidolysis residual rate of the crosslinked gelatin after the irradiation with radiation is 60% or less.

<12> The manufacturing method of a tissue repair material according to any one of <7> to <11>, in which the crosslinked gelatin after the irradiation with radiation is a porous body, and a void ratio of the crosslinked gelatin after the irradiation with radiation is 80.00% to 99.99%.

<13> The manufacturing method of a tissue repair material according to any one of <7> to <12>, in which the gelatin is one or more selected from the group consisting of a peptide consisting of an amino acid sequence set forth in SEQ ID NO: 1, a peptide consisting of an amino acid sequence in which one or several amino acids are deleted, substituted, or added from the amino acid sequence set forth in SEQ ID NO: 1, and having bioaffinity, and a peptide consisting of an amino acid sequence having amino acid sequence identity of 80% or more with a partial amino acid sequence consisting of the 4th to 192nd amino acid residues in the amino acid sequence set forth in SEQ ID NO: 1, and having bioaffinity.

According to an embodiment of the present disclosure, it is possible to provide a tissue repair material having excellent tissue repairability and a manufacturing method of a tissue repair material.

## BRIEF DESCRIPTION OF THE DRAWINGS

Fig. 1 shows an elution curve obtained from the tissue repair material of Example 1.
Fig. 2 shows an elution curve obtained from the tissue repair material of Example 2.
Fig. 3 shows an elution curve obtained from the tissue repair material of Example 3.
Fig. 4 shows an elution curve obtained from the tissue repair material of Example 4.
Fig. 5 shows an elution curve obtained from the tissue repair material of Example 5.
Fig. 6 shows an elution curve obtained from the tissue repair material of Comparative Example 1.

## DESCRIPTION OF THE PREFERRED EMBODIMENTS

Hereinafter, embodiments of the present disclosure will be described in detail. The present disclosure is not limited to the following embodiments. The following embodiments may be modified as appropriate within the scope of the purposes of the present disclosure.

The numerical range indicated by using "to" in the present disclosure indicates a range including numerical value described before as a lower limit and numerical value described after "to" as an upper limit value. Regarding numerical ranges that are described stepwise in the present disclosure, an upper limit value or a lower limit value described in a numerical range may be replaced with an upper limit value or a lower limit value of another stepwise numerical range. In addition, in the numerical ranges described in the present disclosure, an upper limit value and a lower limit value disclosed in a certain range of numerical values may be replaced with values shown in Examples.

In the present disclosure, an amino acid sequence constituting gelatin may be expressed in one-letter notation (for example, "G" in the case of a glycine residue) or three-letter notation (for example, "Gly" in the case of a glycine residue) that is well known in the art.

In the present disclosure, "gelatin" means a polypeptide containing 6 or more amino acid sequences represented by Gly-X-Y in a continuous manner.

In Gly-X-Y, Gly represents a glycine residue and X and Y represent arbitrary amino acid residues other than the glycine residue.

In the present disclosure, the "crosslinked gelatin" means gelatins crosslinked with each other.

In the present disclosure, in a case where a plurality of substances corresponding to each component in a composition is present, the amount of each component in the composition means the total amount of the plurality of substances present in the composition, unless otherwise specified.

In the present disclosure, the term "step" includes not only an independent step but also a step that cannot be clearly distinguished from other steps in a case where a desired object is achieved.

In the present disclosure, the absorption peak of the crosslinked gelatin is confirmed as follows.

First, 5 mg of the tissue repair material is filled into a tube having a capacity of 15 mL, 5 mL of water for injection is added to the tube, and the tube is incubated at 37°C for 24 hours.

1 mL of a supernatant is collected by a low-adsorptive tube after mixing with inversion. The collected solution is filtered through a filter having a pore diameter of 0.22 μm to obtain a measurement sample.

Gel permeation chromatography is carried out on 5 μL of the measurement sample under the following conditions to obtain an elution curve in which the vertical axis is the absorbance and the horizontal axis is the elution time.

A substance having a known molecular weight, such as a polystyrene standard material, is measured at the same or different timing as the measurement sample, and a relationship between the elution time and the molecular weight in the elution curve is understood.

The presence or absence of the absorption peak is confirmed from the elution curve obtained as described above.

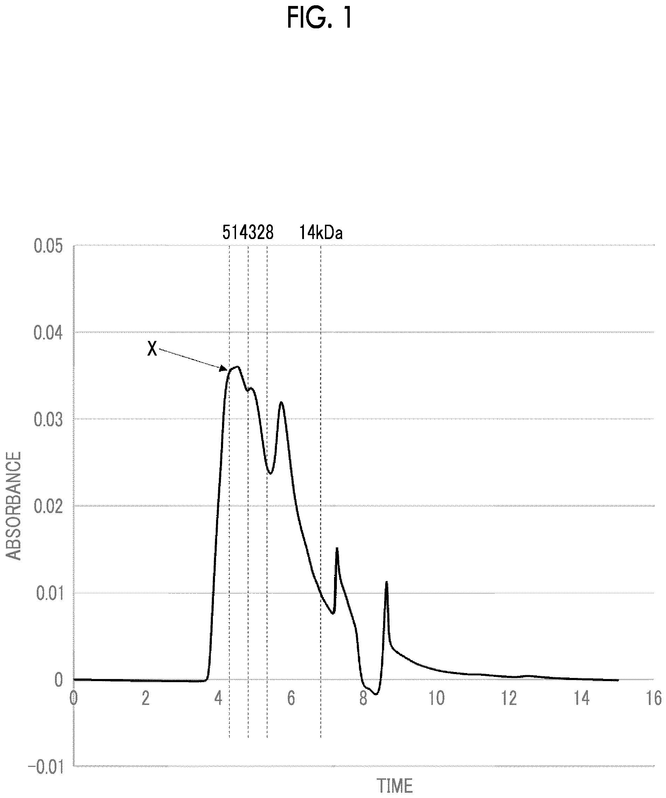

In the present disclosure, the absorption peak includes not only the waveform in which the absorbance is maximal but also the waveform having a shoulder portion indicated by reference numeral X in Fig. 1.

As the column, ACQUITY UPLC Protein BEH SEC Column, 200 Å, 1.7 μm, 4.6 mm × 150 mm or a column having the same performance can be used.

(Conditions)

Column length: 150 mm
Column inner diameter: 4.6 mm

Filler: ethylene bridged (BEH) particles

Particle size of the filler: 1.7 $\mu$m

Pore size of the filler: 200 Å

Column temperature: 30°C

Mobile phase: 0.1 mol/L sodium sulfate, 0.01 mol/L sodium dihydrogen phosphate, and 1% by mass of sodium dodecyl sulfate, pH 5.3

Flow rate: 0.1 mL/minute

Detector: wavelength-tunable UV detector (TUV)

Measuring wavelength: 215 nm

Adjustment of measurement sample: The tissue repair material is filled into a tube having a capacity of 15 mL, 5 mL of water for injection is added to the tube, and the tube is incubated at 37°C for 24 hours. 1 mL of a supernatant is collected by a low-adsorptive tube after mixing with inversion and filtered through a 0.22 $\mu$m filter.

Amount of the measurement sample: 5 $\mu$L

In the present disclosure, the heating temperature of gelatin refers to a setting temperature of an apparatus used for heating, and is not a temperature of the heated gelatin.

In the present disclosure, the water absorption rate of the tissue repair material is measured as follows.

First, a filter cup in which the mass (w0) has been measured is set in a microtube (hereinafter, referred to as a tube).

Next, 500 $\mu$L of water is added to the filter cup, and stirred for 2 hours by a rotator.

After stirring, the tube is centrifuged under the conditions of 25°C and 6,000 $\times$ g for 1 minute, and it is confirmed that water has moved from the filter cup to the inside of the tube. The mass (w1) of the filter cup is measured again, and the residual water amount is calculated using the following expression.

Residual water amount (mg): w1 - w0

Next, another filter cup in which the mass (w2) has been measured is set in the tube, and 10 mg (mass: w3) of the tissue repair material is filled in a filter cup.

Next, 500 $\mu$L of water is added to the filter cup, and stirred for 2 hours by a rotator.

After stirring, the tube is centrifuged under the conditions of 25°C and 6,000 $\times$ g for 1 minute, and it is confirmed that water has moved from the filter cup to the inside of the tube.

The sum (w4) of the mass of the tissue repair material remaining in the filter cup and the mass of the filter cup is measured, and the water absorption rate is calculated using Expression (1).

$$\text{Water absorption rate (\%)} = (w4 - w2 - (w1 - w0))/w3 \times 100 \;....\; (1)$$

In the present disclosure, the acidolysis residual rate of the tissue repair material is measured as follows.

A microtube (hereinafter, referred to as a tube) is prepared, and a mass (A) thereof is measured.

15.0 ($\pm$0.2) mg of the tissue repair material is weighed (mass: B), and is filled in the tube.

1.7 mL of 1 mol/L hydrochloric acid is added to the tube containing the tissue repair material, and the tube is heated for 3 hours using a heat block set at 37°C.

After the heating, the tube is placed on ice to stop the acidolysis reaction, and centrifuged for 1 minute under conditions of 10,000 $\times$ g using a centrifuge set to 4°C in advance.

In the tube, it is confirmed that the tissue repair material is precipitated, the supernatant is suctioned, 1 mL of ultrapure water, which has been cooled on ice in advance, is added thereto, and the mixture is centrifuged again under the same conditions as described above.

After the centrifugation, the supernatant is suctioned, ultrapure water is added to the tube again, and the tube is centrifuged again under the same conditions as described above.

After the centrifugation, the supernatant is suctioned, and then freeze-dried.

After the freeze-drying, the tube is taken out from the freeze-dryer, and the cap of the tube is quickly closed in order to prevent the tissue repair material from absorbing moisture in the air.

The mass (C) of the tube is measured, and the acidolysis residual rate is calculated using Expression (2).

$$\text{Residual rate (\%)} = (C - A)/B \times 100 \;...\; (2)$$

In the present disclosure, the molecular weight of the crosslinked gelatin is a molecular weight converted using polystyrene as a standard substance, and is measured under the above conditions by the above-described gel permeation chromatography.

In the present disclosure, the dose of the radiation is measured by the following method.

A plurality of vials filled with crosslinked gelatin are put into a cardboard box, and a polymethylmethacrylate dosimeter is attached to a side surface of the cardboard box. After the irradiation with radiation in this state, a dose of the radiation measured by the dosimeter is defined as a dose of the radiation applied to the crosslinked gelatin.

[Tissue repair material]

The tissue repair material of the present disclosure contains crosslinked gelatin, and in an elution curve of the crosslinked gelatin, which is obtained by gel permeation chromatography under the following conditions, at least one absorption peak with an absorbance of 0.025 or more is present in a molecular weight range of 14 kDa or more and 51 kDa or less.

(Conditions)

Column length: 150 mm
Column inner diameter: 4.6 mm
Filler: ethylene bridged (BEH) particles
Particle size of the filler: 1.7 $\mu$m
Pore size of the filler: 200 Å
Column temperature: 30°C
Mobile phase: 0.1 mol/L sodium sulfate, 0.01 mol/L sodium dihydrogen phosphate, and 1% by mass of sodium dodecyl sulfate, pH 5.3
Flow rate: 0.1 mL/minute
Detector: wavelength-tunable UV detector (TUV)
Measuring wavelength: 215 nm
Adjustment of measurement sample: The tissue repair material is filled into a tube having a capacity of 15 mL, 5 mL of water for injection is added to the tube, and the tube is incubated at 37°C for 24 hours. 1 mL of a supernatant is collected by a low-adsorptive tube after mixing with inversion and filtered through a 0.22 $\mu$m filter.
Amount of the measurement sample: 5 $\mu$L

In addition, in some cases, the tissue repair material is required that a tissue repair rate (hereinafter, also referred to as a tissue repair speed) after a lapse of about 2 weeks after application to a site where a tissue is deleted or damaged is high. In a case where at least one absorption peak with an absorbance of 0.025 or more is present in a range in a molecular weight range of 14 kDa or more and 51 kDa or less in the elution curve, the tissue repair speed of the tissue repair material tends to be improved.

From the viewpoint of tissue repair speed and tissue repairability, the absorbance of the above-described absorption peak is preferably 0.025 or more, more preferably 0.03 or more, and still more preferably 0.035 or more.

The upper limit of the absorbance of the above-described absorption peak is not particularly limited, and may be 0.06 or less.

From the viewpoint of tissue repair speed and tissue repairability, it is preferable that two or more absorption peaks are present, and it is more preferable that three or more absorption peaks are present.

In a case where two or three absorption peaks are present, it is preferable that the absorption peaks are present both in a molecular weight range of 14 kDa or more and less than 28 kDa and in a molecular weight range of 28 kDa or more and less than 43 kDa, and it is preferable that the absorption peaks are present both in a molecular weight range of 43 kDa or more and 51 kDa or less, in a molecular weight range of 28 kDa or more and less than 43 kDa, and in a molecular weight range of 14 kDa or more and less than 28 kDa.

From the viewpoint of tissue repair speed and tissue repairability, in the elution curve of the crosslinked gelatin, a ratio of an area at a molecular weight of 14 kDa or more and 43 kDa or less to an area at a molecular weight of more than 43 kDa and 51 kDa or less is preferably 2.5 or more, more preferably 3.0 or more, and still more preferably 7.0 or more.

The upper limit of the above-described area ratio is not particularly limited, and may be 30.00 or less.

In the present disclosure, the area of the molecular weight of more than 43 kDa and 51 kDa or less and the area of the molecular weight of 14 kDa or more and 43 kDa or less are determined from the elution curve obtained as described above.

Specifically, in each of a molecular weight range of more than 43 kDa and 51 kDa or less or in a molecular weight range of 14 kDa or more and 43 kDa or less, the area is determined by obtaining an area of the region surrounded by the elution curve and the baseline.

The baseline refers to a straight line having an absorbance of 0.

The water absorption rate of the tissue repair material of the present disclosure is preferably 450% or more, more preferably 500% or more, and still more preferably 600% or more.

In a case where the water absorption rate of the tissue repair material is 450% or more, the blood clot retentivity during tissue repair can be improved, and the tissue repair speed and the tissue repairability can be improved.

The upper limit value of the water absorption rate of the tissue repair material is not particularly limited, but is preferably 9,900% or less, more preferably 5,000% or less, and still more preferably 3,000% or less.

The water absorption rate of the tissue repair material can be adjusted by the components contained in the tissue repair material, the type of the crosslinked gelatin, and the form of the crosslinked gelatin. In addition, the water absorption rate can be adjusted by the temperature of the freezing step and the crosslinking step, the crosslinking treatment time of the crosslinking step, and the like in the manufacturing method of the tissue repair material.

In general, in a case where the temperature of the freezing step is increased, the temperature of the crosslinking step is decreased, or the crosslinking treatment time of the crosslinking step is shortened, the water absorption rate tends to increase.

From the viewpoints of tissue repair speed and tissue repairability, in the tissue repair material of the present disclosure, a residual rate (acidolysis residual rate) by the decomposition treatment using 1 mol/liter (L) of hydrochloric acid for 3 hours is preferably 60% by mass or less, more preferably 55% by mass or less, and still more preferably 50% by mass or less.

In addition, from the viewpoints of maintaining the volume at the defective portion and substituting with the tissue to be regenerated, the acidolysis residual rate of the tissue repair material is preferably 5% by mass or more, more preferably 20% by mass or more, and still more preferably 40% by mass or more.

The acidolysis residual rate of the tissue repair material can be adjusted by the components contained in the tissue repair material, the type of the crosslinked gelatin, and the form of the crosslinked gelatin. In addition, the acidolysis residual rate can be adjusted by the temperature of the freezing step and the crosslinking step, the crosslinking treatment time of the crosslinking step, and the like in the manufacturing method of the tissue repair material.

In general, in a case where the temperature of the freezing step is increased, the temperature of the crosslinking step is decreased, or the crosslinking treatment time of the crosslinking step is shortened, the acidolysis residual rate tends to decrease.

<Crosslinked gelatin>

The tissue repair material of the present disclosure contains crosslinked gelatin obtained by performing a heat treatment, a crosslinking agent treatment, or the like on gelatin.

From the viewpoints of tissue repair speed and tissue repairability, the crosslinked gelatin is preferably obtained by performing a heat treatment on gelatin, and in the present disclosure, the crosslinked gelatin which has been subjected to the heat treatment is also referred to as dehydrated crosslinked gelatin. The dehydrated crosslinked gelatin does not require a crosslinking agent in the crosslinking, or the amount of the crosslinking agent used is small, and the dehydrated crosslinked gelatin has a low potential to cause cytotoxicity, inflammation, or the like, thereby the dehydrated crosslinked gelatin is preferable from the viewpoint of biological safety.

The gelatin used for manufacturing the crosslinked gelatin may be a natural type gelatin or a variant type or recombinant thereof in which at least one amino acid residue is different from the natural type.

In the present disclosure, the natural type gelatin means gelatin produced from natural collagen as a raw material or a polypeptide having the same amino acid sequence as gelatin produced from natural collagen as a raw material.

Unless otherwise noted, in the present disclosure, variant type gelatin or recombinant thereof are collectively referred to as recombinant gelatin.

Examples of the natural type gelatin or the recombinant gelatin thereof include gelatin derived from animals such as fish and mammals, and a natural type gelatin derived from mammals or the recombinant gelatin thereof is preferable.

Examples of the mammal include a human, a horse, a pig, a mouse, a rat, and the like, and a human or a pig is more preferable.

The natural type gelatin is preferably pig-derived natural type gelatin or human-derived natural type gelatin, and the recombinant gelatin is preferably human-derived recombinant gelatin.

In the present disclosure, the gelatin means a polypeptide including 6 or more of the amino acid sequences represented by Gly-X-Y in a continuous manner, and may have one or more other amino acid residues in addition to the amino acid sequence represented by Gly-X-Y in the polypeptide. In Gly-X-Y, Gly represents a glycine residue and X and Y represent arbitrary amino acid residues other than the glycine residue.

The amino acid sequence represented by Gly-X-Y is a sequence corresponding to an amino acid sequence derived from a partial amino acid sequence of collagen, and the repetition of this sequence means a sequence characteristic of collagen.

A plurality of Gly-X-Y's in one molecule of gelatin may be the same or different from each other. In addition, X and Y in the Gly-X-Y sequence are independent for each repeating unit, and may be the same or different from each other.

As X and Y, a large number of imino acid residues (specifically, a proline residue or an oxypyrroline residue) are preferably contained. The content of such an imino acid residue is preferably 10% by mass to 45% by mass in one molecule of gelatin. A content of Gly-X-Y in one molecule of gelatin is preferably 80% by mass or more, more preferably 95% by mass or more, and most preferably 99% by mass or more of the total.

The gelatin is preferably a recombinant gelatin obtained by transferring and expressing, in an appropriate host, a gene having a base sequence or an amino acid sequence in which one or more bases or amino acid residues are changed with respect to the base sequence or the amino acid sequence encoding collagen having 6 or more amino acid sequences represented by Gly-X-Y in a continuous manner, with a usual method.

By using such a recombinant gelatin, it is possible to increase the tissue repair speed and the tissue repairability, and to exhibit various characteristics as compared with a case of using natural type gelatin. For example, it is possible to avoid an adverse effect such as a rejection reaction in a living body.

As the recombinant gelatin, those disclosed in EP1014176A2, US6992172B1, WO2004/85473A2, WO2008/103041A1, JP2010-519293A, JP2010-519252A, JP2010-518833A, JP2010-519251A, WO2010/128672A1, WO2010/147109A1, or the like can be particularly preferably used.

The molecular weight of the gelatin is preferably 2 kDa to 100 kDa, more preferably 5 kDa to 90 kDa, and still more preferably 10 kDa to 90 kDa.

From the viewpoint of bioaffinity, the gelatin preferably further includes a cell adhesion signal, and more preferably has two or more cell adhesion signals in one molecule.

Examples of the cell adhesion signal can include each of an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence, and an RGD sequence, a YIGSR sequence, a PDSGR sequence, an LGTIPG sequence, an IKVAV sequence, or an HAV sequence is preferable and an RGD sequence is more preferable. It is still more preferable that such the cell adhesion signal is ERGD sequence among the RGD sequences.

An arrangement of the RGD sequence in the gelatin, the number of amino acid residues between the RGD sequences is preferably 0 to 100, and more preferably 25 to 60. In addition, it is preferable that the RGD sequence is unevenly arranged within the above-described range of the number of amino acid residues.

The proportion of the RGD sequence to the total number of amino acid residues in gelatin (total number of amino acid residues in RGD sequence/total number of amino acid residues × 100) is preferably at least 1.2%, and in a case where the recombinant gelatin includes 250 or more amino acid residues, it is preferable that each stretch of 250 amino acid residues includes at least one RGD sequence.

The gelatin more preferably includes at least two RGD sequences, more preferably includes at least three RGD sequences, and still more preferably includes at least four RGD sequences, per 250 amino acid residues.

The arrangement of the gelatin preferably satisfies at least one of the following aspects, but the present invention is not limited thereto.

(1A) a sequence not including a serine residue and a threonine residue,
(2A) a sequence not including a serine residue, a threonine residue, an asparagine residue, a tyrosine residue, and a cysteine residue, and
(3A) a sequence not including the amino acid sequence represented by Asp-Arg-Gly-Asp.

The gelatin may be partially hydrolyzed.

The gelatin preferably has an amino acid sequence of A-[(Gly-X-Y)$_n$]$_m$-B. Here, A represents one or two or more of any amino acid residues, B represents one or two or more of any amino acid residues, Gly represents a glycine residue, n pieces of X's each independently represent any amino acid residue, and n pieces of Y's each independently represent any amino acid residue.

m represents an integer of 2 to 10, and preferably represents an integer of 3 to 5. n represents an integer of 3 to 100, preferably an integer of 15 to 70, and more preferably an integer of 50 to 65. m pieces of Gly-X-Y's may all be the same, may be partially the same, or may be different from each other.

More preferably, the recombinant gelatin has an amino acid sequence of Gly-Ala-Pro-[(Gly-X-Y)$_{63}$]$_3$-Gly.

Here, 63 X's each independently represent any amino acid residue, and 63 Y's each independently represent any amino acid residue. All of the 63 Gly-X-Y's may be the same, a part of the 63 Gly-X-Y's may be the same, or the 63 Gly-X-Y's may be different from each other.

It is preferable that a repeating unit of the gelatin is formed by bonding, in a case where a part of an amino acid sequence of naturally occurring collagen is defined as one unit, a plurality of the units. Preferred examples of the naturally occurring collagen referred to herein include type I collagen, type II collagen, type III collagen, type IV collagen, or type V collagen, and type I collagen, type II collagen, or type III collagen is more preferable.

The collagen is preferably collagen derived from a human, a horse, a pig, a mouse, or a rat, and more preferably collagen derived from a human.

The isoelectric point of the gelatin is preferably 5 to 10, more preferably 6 to 10, and still more preferably 7 to 9.5.

The isoelectric point of gelatin is calculated based on the amino acid composition of gelatin.

The gelatin preferably satisfies at least one of the following aspects, but the present invention is not limited thereto.

(1B) the carbamoyl group has not been hydrolyzed,
(2B) not having procollagen,
(3B) not having a teropeptide, and
(4B) a substantially pure collagen-like material prepared from a nucleic acid encoding natural collagen.

From the viewpoints of tissue repair speed and tissue repairability, the gelatin is preferably one or more selected from the group consisting of a peptide (hereinafter, also referred to as a specific peptide A) consisting of an amino acid sequence set forth in SEQ ID NO: 1, a peptide (hereinafter, also referred to as a specific peptide B) consisting of an amino acid sequence (hereinafter, also referred to as a specific amino acid sequence B) in which one or several amino acids are deleted, substituted, or added from the amino acid sequence set forth in SEQ ID NO: 1, and having bioaffinity, and a peptide (hereinafter, also referred to as a specific peptide C) consisting of an amino acid sequence (hereinafter, also referred to as a specific amino acid sequence C) having amino acid sequence identity of 80% or more with the partial amino acid sequence consisting of the 4th to 192nd amino acid residues in the amino acid sequence set forth in SEQ ID NO: 1, and having bioaffinity.

GAP(GAPGLQGAPGLQGMPGERGAAGLPGPKGERGDAGPKGADGAPGAPGLQGMPG

ERGAAGLPGPKGERGDAGPKGADGAPGKDGVRGLAGPIGPPGERGAAGLPGPKGER

GDAGPKGADGAPGKDGVRGLAGPIGPPGPAGAPGAPGLQGMPGERGAAGLPGPKGE

RGDAGPKGADGAPGKDGVRGLAGPP)$_3$G (SEQ ID NO: 1)

In the specific peptide B, the number of amino acid residues to be deleted, substituted, or added may be one or several, can be 2 to 15, and is preferably 2 to 5.

From the viewpoints of tissue repair speed and tissue repairability, the sequence identity of the specific peptide C is preferably 90% or more and more preferably 95% or more.

The partial amino acid sequence is a partial amino acid sequence corresponding to a repeating unit of the sequence set forth in SEQ ID NO: 1.

The specific peptide C may include two or more of the specific amino acid sequences C.

A proportion of the number of amino acid residues included in the specific amino acid sequence C to the total number of amino acid residues included in the specific peptide C is preferably 80% or more.

The recombinant gelatin can be manufactured by a gene recombination technique known to those skilled in the art.

For example, the recombinant gelatin can be manufactured according to the method described in EP1014176A2, US6992172B1, WO2004/85473A2, WO2008/103041A1, or the like.

The number of amino acid residues included in the gelatin can be 151 to 2,260, from the viewpoint of decomposability after crosslinking, is preferably 193 or more, and from the viewpoint of stability, is preferably 944 or less and more preferably 380 to 756.

The form of the crosslinked gelatin is not particularly limited, and examples thereof include a form such as a sponge, a film, a nonwoven fabric, particles, and a mesh.

Among these, from the viewpoints of cell invasiveness, tissue repair speed, and tissue repairability, the form of the crosslinked gelatin is preferably particles (hereinafter, the particulate crosslinked gelatin is also referred to as crosslinked gelatin particles).

From the viewpoint of cell invasiveness, the crosslinked gelatin particles are preferably particles that pass through a 4 mm opening sieve, more preferably particles that pass through a sieve having an opening size of 1,400 μm, still more preferably particles that pass through a sieve having an opening size of 1,000 μm, and particularly preferably a particle group that passes through a sieve having an opening size of 710 μm.

From the viewpoint of elasticity of a layer (hereinafter, also referred to as a formulation layer) formed by disposing the tissue repair material of the present disclosure in a predetermined space, the crosslinked gelatin particles preferably remain on a sieve having an opening size of 75 μm and more preferably remain on a sieve having an opening size of 300

μm.

For the sieving of the crosslinked gelatin particles, an ISO3310 standard test sieve is used, and the sieving method is according to the sieving method described in the second method of section 3.04 of the 16th revised Japanese Pharmacopoeia.

That is, an operation of shaking the granules for 5 minutes is intermittently performed a plurality of times, and at a point in time when the ratio of the mass of a group of particles remaining on the sieve after the shaking to the mass of a group of particles on the sieve before the shaking becomes equal to or lower than 5%, the operation is ended.

In the present disclosure, the term "pass" means that the particles remaining on the sieve at the above-described end of the operation are 10% by mass or less of the total mass of the particles before the sieving. In addition, in the present disclosure, the term "remain" means that the particles remaining on the sieve at the above-described end of the operation is 95% by mass or more of the total mass of the particles before the sieving.

From the viewpoints of tissue repair speed and tissue repairability, the crosslinked gelatin is preferably a porous body.

From the viewpoints of cell invasiveness, tissue repair speed, and tissue repairability, the void ratio of the crosslinked gelatin is preferably 80% to 99.99% and more preferably 95.01% to 99.9%.

In the present disclosure, the void ratio of the crosslinked gelatin is obtained by using the bulk density ($\rho$) of the crosslinked gelatin and the true density ($\rho c$) of the crosslinked gelatin according to the following expression.

The bulk density ($\rho$) is calculated from the dried mass and volume of the crosslinked gelatin, and the true density ($\rho c$) of the crosslinked gelatin is obtained by the pycnometer method using a Guy-Lus sac-type pycnometer.

$$\text{Void ratio P (\%)} = (1 - \rho/\rho c) \times 100$$

From the viewpoint of cell invasiveness, the void ratio of the formulation layer formed by disposing the tissue repair material of the present disclosure in a predetermined space is preferably 70% to 96.5% and more preferably 80% to 90%.

In the present disclosure, the void ratio of the formulation layer is obtained from the following expression by using the tap density ($\rho t$) of the crosslinked gelatin particles and the true density ($\rho c$) of the crosslinked gelatin particles.

$$\text{Void ratio P (\%) of the formulation layer} = (1 - \rho t/\rho c) \times 100$$

The tap density ($\rho t$) is obtained by a method described below. The true density ($\rho c$) is obtained by the pycnometer method using a Hubbard-type pycnometer.

The crosslinked gelatin particles may have communication holes. In a case where the crosslinked gelatin particles have the communication holes, the voids extend from the outside to the inside of the tissue repair material, and thus the cells in contact with the outside of the tissue repair material can disperse or diffuse into the inside of the tissue repair material.

The pore diameter of the communication hole is preferably 10 μm to 2,500 μm, more preferably 50 μm to 2,500 μm, and still more preferably 100 μm to 1,000 μm to exhibit the above-described function.

From the viewpoints of elasticity of the formulation layer and cell invasiveness, the tap density of the crosslinked gelatin particles is preferably 10 mg/cm$^3$ to 500 mg/cm$^3$, more preferably 30 mg/cm$^3$ to 450 mg/cm$^3$, still more preferably 50 mg/cm$^3$ to 420 mg/cm$^3$, and particularly preferably 140 mg/cm$^3$ to 280 mg/cm$^3$.

In the present disclosure, the tap density is a value indicating how many particles can be densely filled in a certain volume, and the smaller the value is, the more complicated the structure of the formulation layer tends to be, the wider the particle size distribution also tends to be, and the more the particles tend to be coarsely filled.

In the present disclosure, the tap density is measured by the following method.

First, a cylindrical container (volume: 0.616 cm$^2$) having a diameter of 6 mm and a length of 21.8 mm (hereinafter, referred to as a cap) is prepared, and the mass of only the cap is measured (wt).

Next, the cap and the funnel are connected to each other, and the crosslinked gelatin particles are poured from the neck such that the crosslinked gelatin particles are accumulated in the cap.

After pouring a sufficient amount of the crosslinked gelatin particles, the cap portion is tapped 200 times on a hard place such as a desk, the funnel is removed, and the crosslinked gelatin particles that are raised beyond the edge of the cap are scraped off with a spatula.

The mass of the crosslinked gelatin particles in a state of being filled with one level cup is measured (wg).

The mass of only the crosslinked gelatin particles is calculated from the difference between the mass wt and the mass wg, which has been measured as described above, and the tap density is obtained by dividing the mass of only the crosslinked gelatin particles by the volume of the cap.

$$\text{Tap density } \rho t = (wg - wt)/0.616$$

From the viewpoints of tissue repair speed and tissue repairability, the content of the crosslinked gelatin with respect to the total mass of the tissue repair material of the present disclosure is preferably 70% by mass or more, more preferably 80% by mass or more, still more preferably 90% by mass or more, and particularly preferably 95% by mass or more.

The upper limit of the content of the crosslinked gelatin is not particularly limited, and may be 100% by mass.

<Component other than crosslinked gelatin>

The tissue repair material according to the embodiment of the present disclosure may contain a component other than the crosslinked gelatin, and examples of the component include a component related to bone regeneration or bone neoplasticity, such as a bone-inducing agent. Examples of the bone-inducing agent include a bone morphogenetic protein (BMP), a basic fibroblast growth factor (bFGF), and the like.

[Manufacturing method of tissue repair material]

The manufacturing method of a tissue repair material of the present disclosure includes a step of heating gelatin under conditions of 100°C to 170°C and 2 hours to 24 hours to obtain crosslinked gelatin (hereinafter, referred to as a crosslinking step), and a step of irradiating the crosslinked gelatin with radiation having a dose of 5 kGy to 50 kGy (hereinafter, referred to as a radiation irradiating step).

The manufacturing method of the tissue repair material according to the present disclosure may include a step of preparing a gelatin solution containing gelatin dissolved in an aqueous medium before the step of obtaining the crosslinked gelatin (hereinafter, referred to as a gelatin solution preparation step), a step of freeze-drying the gelatin solution to obtain a freeze-dried body (hereinafter, referred to as a freeze-drying step), and a step of pulverizing the freeze-dried body to obtain a pulverized product (hereinafter, referred to as a pulverizing step).

In addition, the manufacturing method of the tissue repair material of the present disclosure preferably includes, before the freeze-drying step, a step of cooling the gelatin solution to a temperature lower than an ice crystal formation temperature (hereinafter, referred to as an ice crystal formation step) to obtain the crosslinked gelatin which is a porous body.

<Crosslinking step>

The manufacturing method of a tissue repair material of the present disclosure includes a step of heating gelatin under conditions of 100°C to 170°C and 2 hours to 24 hours to obtain crosslinked gelatin.

The type of the gelatin to be crosslinked, and the like are as described above, and thus the description thereof will be omitted here. In addition, the gelatin to be crosslinked may be a freeze-dried body pulverized product of a gelatin solution, which will be described later.

From the viewpoints of tissue repair speed and tissue repairability, the heating temperature is preferably 120°C to 170°C, more preferably 120°C to 150°C, and still more preferably 130°C to 140°C.

From the viewpoints of tissue repair speed and tissue repairability, the heating time is preferably 2 hours to 20 hours, more preferably 4 hours to 12 hours, and still more preferably 4.5 hours to 8 hours.

The heating of gelatin is, in order to prevent oxidation, preferably performed under reduced pressure, in a vacuum, or in an inert gas atmosphere, more preferably performed in a vacuum or in an inert gas atmosphere, and still more preferably performed in an inert gas atmosphere.

In a case where the gelatin is heated under reduced pressure, it is preferable to set the environment to 4 hPa or less.

The inert gas is preferably nitrogen.

For heating the gelatin, a known heating device in the related art can be used, and for example, DP-43 manufactured by Yamato Scientific Co., Ltd. can be used.

<Radiation irradiating step>

Examples of the radiation used in the radiation irradiating step include $\alpha$-rays, $\beta$-rays, $\gamma$-rays, neutron rays, electron beams, and X-rays, and among these, from the viewpoints of tissue repair speed and tissue repairability, $\gamma$-rays or electron beams are preferable, and $\gamma$-rays are more preferable.

From the viewpoints of tissue repair speed and tissue repairability, the irradiation dose of the radiation is preferably 10 kGy to 48 kGy, more preferably 12 kGy to 45 kGy, and still more preferably 12 kGy to 30 kGy.

The preferred numerical ranges of the irradiation dose and the irradiation time are preferred numerical ranges in a case where the crosslinked gelatin is irradiated with radiation from one direction, and in a case where the crosslinked gelatin is irradiated from two or more directions, the preferred numerical ranges are not limited to the above, and are preferably adjusted as appropriate.

<Gelatin solution preparation step>

In the gelatin solution preparation step, the gelatin solution may be prepared by dissolving gelatin in an aqueous medium, or a prepared gelatin solution may be prepared.

The aqueous medium that can be used is not particularly limited as long as the aqueous medium can dissolve a gelatin and can be used for biological tissue. Examples thereof can include water, saline, and a phosphate buffer solution, and the like.

A content of gelatin with respect to the total mass of the gelatin solution is not particularly limited, and is preferably 0.5% by mass to 20% by mass, more preferably 2% by mass to 16% by mass, and still more preferably 4% by mass to 12% by mass.

In a case where the content of the gelatin is 0.5% by mass or more, the strength of the tissue repair material tends to be increased, and in a case where the content of the gelatin is 20% by mass or less, the tissue repair material tends to form a mesh structure with high uniformity, and the tissue repair speed and the tissue repairability tend to be improved.

A temperature of the aqueous medium in a case of preparing the gelatin solution can be 0°C to 60°C, and is preferably 3°C to 30°C.

The gelatin solution may contain a component other than gelatin, such as a crosslinking agent.

<Freeze-drying step>

In the freeze-drying step, the gelatin solution is freeze-dried to obtain a freeze-dried body. In a case where the manufacturing method of the tissue repair material of the present disclosure includes the ice crystal formation step, the gelatin solution after the cooling is freeze-dried.

As the freezing condition, the condition generally used for freeze-drying a protein may be used as it is. The freeze-drying time can be, for example, 0.5 hours to 300 hours. The available freeze dryer is also not particularly limited.

<Pulverizing step>

In the pulverizing step, the freeze-dried body of gelatin is pulverized to obtain a pulverized product. The pulverization can be performed by using a pulverizer such as a hammer mill or a screen mill.

From the viewpoint that the pulverized product pulverized to a constant size can be collected at any time and the variation in particle diameter is small, it is preferable to use a screen mill. As the screen mill, a co-mill manufactured by Quadro Engineering Corp. or the like can be used.

Examples of the pulverizing method include a crushing method, a cutting method, and the like.

The pulverizing step may include pulverizing the freeze-dried body of gelatin to obtain a pulverized product, and then classifying the pulverized product. As a result, a pulverized product having a uniform particle diameter can be obtained.

For the classification, for example, it is preferable to use a sieve having an opening size of 300 $\mu$m to 1,400 $\mu$m.

<Ice crystal formation step>

The manufacturing method of the tissue repair material of the present disclosure can include an ice crystal formation step before the freeze-drying step. As a result, it is possible to obtain the gelatin-containing intermediate having an ice crystal inside.

Since the formed ice crystal causes roughening of the peptide chain of gelatin and solidification of the gelatin-containing intermediate, the gelatin-containing intermediate having a void inside is formed after the ice crystal disappears. The disappearance of the ice crystal can be performed by drying in the freeze-drying step.

The pore diameter of the void in the gelatin-containing intermediate can be adjusted by an ice crystal temperature, a cooling time, and the like.

The shape of the void is not particularly limited, and may be a two-dimensional structure or a three-dimensional structure. A cross-sectional shape of the mesh is not particularly limited, and examples thereof include a polygon, a circle, an ellipse, and the like. Examples of the three-dimensional structure of the void include a columnar structure, a spherical structure, and the like. From the viewpoints of tissue repair speed and tissue repairability, the shape of the void is preferably spherical.

The gelatin-containing intermediate may have a communication hole in which voids are continuously formed. Since the communication hole has been described above, the description thereof will be omitted here.

From the viewpoints of tissue repair speed and tissue repairability, the pore diameter of the void is preferably 10 $\mu$m or more, more preferably 50 $\mu$m or more, and still more preferably 100 $\mu$m or more. The upper limit of the pore diameter is not particularly limited, but from the viewpoints of substance strength stability and bioaffinity, 2,500 $\mu$m or less is preferable and 1,000 $\mu$m or less is more preferable.

The pore diameter of the void is an average of the diameter (long axes) in the major axis direction, and is measured as follows.

First, a dried intermediate is obtained by drying the gelatin-containing intermediate is prepared, and a test piece obtained by cutting the dried intermediate in the horizontal direction and a test piece obtained by cutting the dried intermediate in the vertical direction are prepared.

The horizontal direction of the dried intermediate is a direction horizontal to a surface of the dried intermediate in a case where the dried intermediate is allowed to stand on a flat surface. The dried intermediate is allowed to stand such that an area in contact with a flat surface is maximized.

Next, the cross section of each test piece is dyed by closely attaching the cross section to a stamping table, and a region of 2.0 mm $\times$ 2.0 mm is observed with an optical microscope.

Among rectangles circumscribed about the region (one void) in the observation region surrounded by the dyed material, a circumscribed rectangle in which the distance between two opposing sides of the rectangle is largest is selected.

In each of the observation region of the cross section of the test piece cut in the horizontal direction and the observation region of the cross section of the test piece cut in the vertical direction, 50 lengths of the long side of the circumscribed rectangle in which the distance between two opposing sides of the rectangle is largest are measured, and the average thereof is defined as the average value of the major axis of the void of the gelatin-containing intermediate.

A smaller one of an average value of major axis of a cross section of a test piece cut in a horizontal direction and an average value of major axis of a cross section of a test piece cut in a vertical direction, which are obtained by the above-described method, is defined as d1, and the other is defined as d2, and a ratio d2/d1 is defined as an aspect ratio.

In the present disclosure, a case in which the aspect ratio of the void is 1 to 3 is referred to as "spherical", and a case outside this range is referred to as "columnar".

In a case where the shape of the void is columnar, from the viewpoints of tissue repair speed and tissue repairability, the aspect ratio is preferably 4 or 5.

The void ratio of the gelatin-containing intermediate is preferably 80% to 99.99% and more preferably 95.01% to 99.9%.

In the present disclosure, the void ratio of the gelatin-containing intermediate is obtained by using the bulk density ($\rho$1) of the gelatin-containing intermediate, the true density ($\rho$c1) of the crosslinked gelatin, and the following expression.

The bulk density ($\rho$1) is calculated from the dry mass and volume of the gelatin-containing intermediate, and the true density ($\rho$c1) of the gelatin-containing intermediate is obtained by the pycnometer method using a Guy-Lus sac-type pycnometer.

$$\text{Void ratio P1 (\%)} = (1 - \rho1/\rho c1) \times 100$$

The ice crystal formation temperature means a temperature at which at least a part of the gelatin solution is frozen.

The ice crystal formation temperature varies depending on the concentration of solid contents of the gelatin solution, but is generally -10°C or less.

The cooling temperature of the gelatin solution is preferably -100°C to -10°C, more preferably -80°C to -20°C, and still more preferably -40°C to -60°C.

By setting the cooling temperature of the gelatin solution to -100°C or more, the void has a sufficient size, and the tissue repair speed and the tissue repairability of the tissue repair material tend to be improved.

By setting the cooling temperature to -10°C or less, the uniformity of the void pore diameter is high, and the tissue repair speed and the tissue repairability tend to be improved.

From the viewpoint that the ice crystal formation is likely to occur uniformly, the cooling time is preferably 1 hour to 6 hours.

[Use of tissue repair material]

The use of the tissue repair material of the present disclosure includes applying the tissue repair material to a site where a tissue is deleted or damaged in a target tissue.

Target tissues are preferably hard tissues such as teeth and bones, and the tissue repair material is suitable for bone regeneration.

The use of the tissue repair material of the present disclosure may include, as necessary, applying at least one of the transplant cells or the bone inductor to a site to be applied with the tissue repair material before or after the application of the tissue repair material.

In a treatment method or a repair method, the use of the tissue repair material can be preferably applied to a periodontal defect, an implant defect, or the like in the maxillofacial region; a GBR method, a ridge augmentation method, a sinus lift method, a socket reservation method, or the like as a preliminary treatment at the time of implant embedding.

Examples

Hereinafter, the present disclosure will be described in detail with reference to Examples. However, the present disclosure is not limited to the following Examples.

(Example 1)

As the recombinant gelatin, CBE3 described in WO2008/103041A1 was prepared.

The details of the CBE3 are as follows.

· Molecular weight: 51.6 kDa
· Number of amino acid residues: 571
· Number of RGD sequences: 12
· Amino acid sequence: SEQ ID NO: 1

GAP(GAPGLQGAPGLQGMPGERGAAGLPGPKGERGDAGPKGADGAPGAPGL

QGMPGERGAAGLPGPKGERGDAGPKGADGAPGKDGVRGLAGPIGPPGERGAAGLPG

PKGERGDAGPKGADGAPGKDGVRGLAGPIGPPGPAGAPGAPGLQGMPGERGAAGLP

GPKGERGDAGPKGADGAPGKDGVRGLAGPP)$_3$G (SEQ ID NO: 1)

An aqueous solution containing 7.5% by mass of CBE3 was prepared.

The above-described aqueous solution was poured into a cylindrical container and then placed in a freeze dryer.

The aqueous solution was frozen at -60°C for 1 hour or more, and then subjected to primary drying at -15°C for 38 hours and secondary drying at 23°C for 2 hours under reduced pressure to obtain a sponge-like gelatin.

The sponge-like gelatin was pulverized with a screen pulverizer (Co-Mill U10, manufactured by Quadro Engineering Corp.) using a 0.079 inch screen and then a 0.040 inch screen to obtain a pulverized product.

Using a test sieve having an ISO 3310 standard, the pulverized product was sieved according to the method described in the second method of section 3.04 of the 16th revised Japanese Pharmacopoeia.

Specifically, the fraction that passed through a sieve having an opening size of 1400 μm and remained on a sieve having an opening size of 300 μm was collected was collected, and filled in an amount of about 0.09 g in a 10 mL glass vial (φ24.3 mm, height 46.5 mm).

The pulverized product filled in the glass vial was placed in a clean oven (manufactured by Nitto Rika Kogyo Co. Ltd., NCO-500A600L-WS), and the pulverized product was heated in a nitrogen atmosphere at a heating temperature of 135°C to 140°C and a heating time of 4.75 hours to obtain particulate crosslinked gelatin. Subsequently, the glass vial was capped with a rubber stopper (rubber stopper 2A), and seamed with an aluminum cap.

The glass vial bottle filled with the crosslinked gelatin was put into a cardboard box, a polymethylmethacrylate dosimeter was attached to a side surface of the cardboard box, and the dosimeter was irradiated with a dose of 18 kGy to obtain a tissue repair material.

(Example 2)

A tissue repair material was obtained in the same manner as in Example 1, except that the dose of the irradiated γ-rays was changed to 28 kGy.

(Example 3)

A tissue repair material was obtained in the same manner as in Example 1, except that the dose of the irradiated $\gamma$-rays was changed to 13 kGy and the heating time of the pulverized product was changed to 6.25 hours.

(Example 4)

A tissue repair material was obtained in the same manner as in Example 1, except that the dose of the irradiated $\gamma$-rays was changed to 28 kGy and the heating time of the pulverized product was changed to 6.25 hours.

(Example 5)

A tissue repair material was obtained in the same manner as in Example 1, except that the dose of the irradiated $\gamma$-rays was changed to 28 kGy and the heating time of the pulverized product was changed to 8.00 hours.

(Comparative Example 1)

A tissue repair material was obtained in the same manner as in Example 1, except that the $\gamma$-ray irradiation was not performed.

<<Acquisition of elution curve by gel permeation chromatography>>

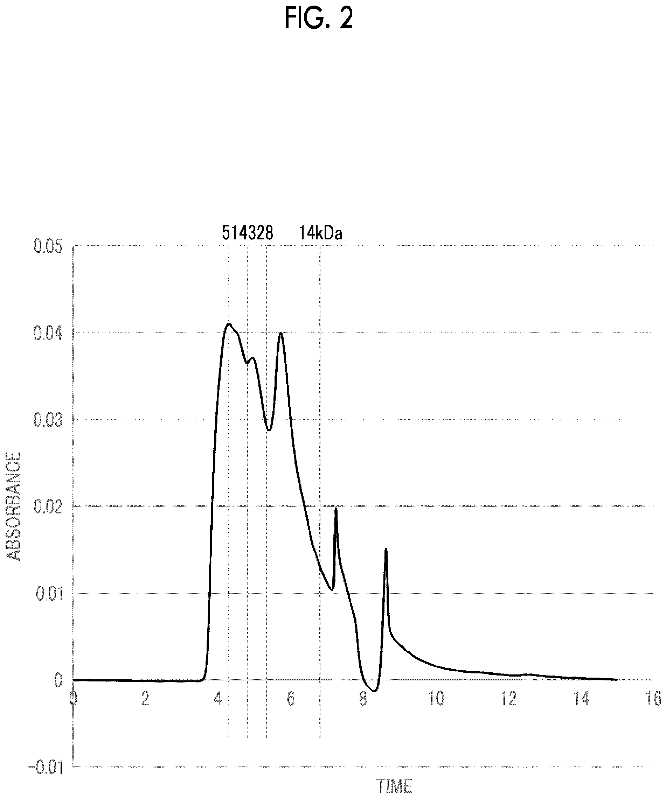

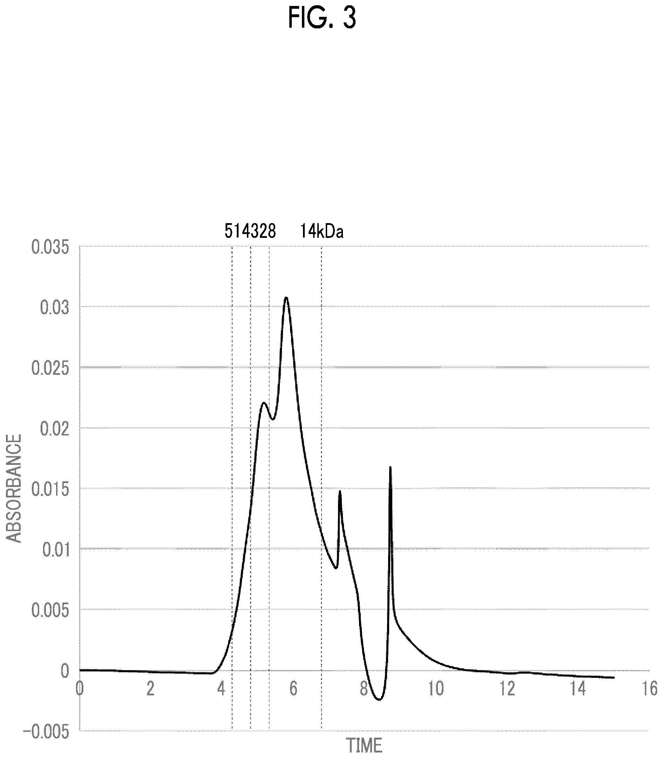

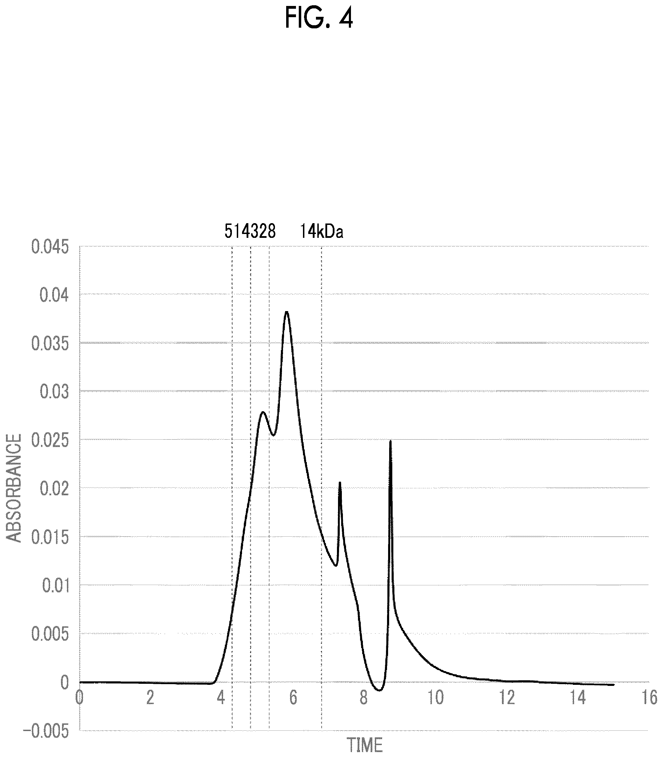

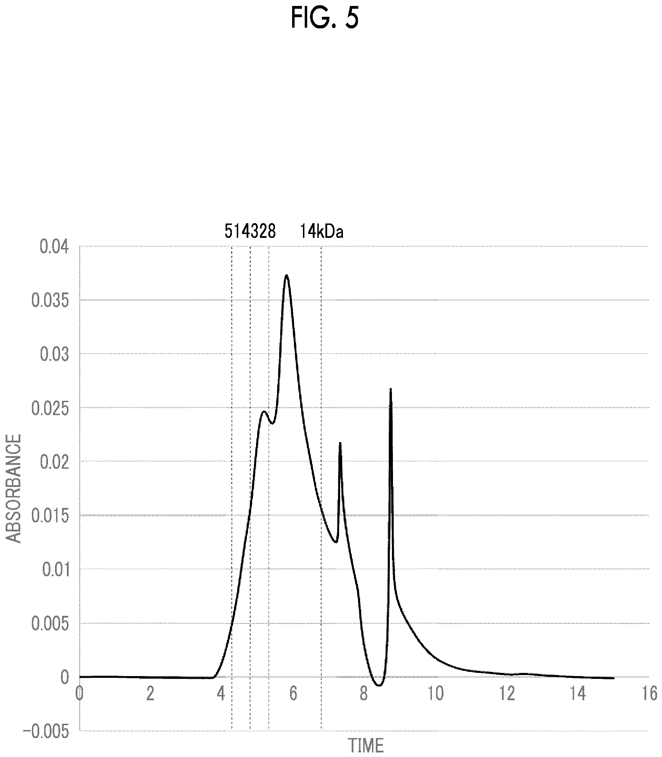

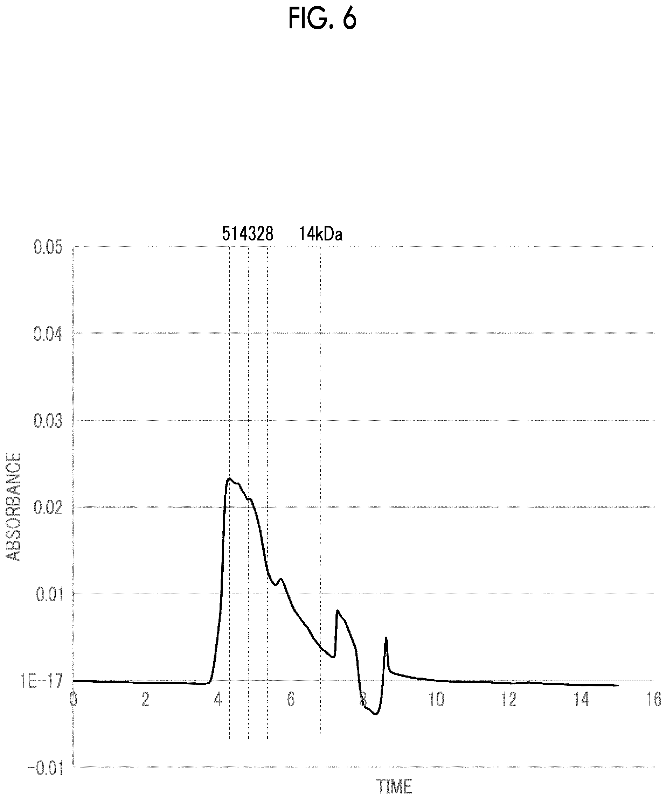

The tissue repair material manufactured in Examples and Comparative Examples was charged into a tube having a capacity of 15 mL, 5 mL of distilled water for injection was added to the tube, and the mixture was incubated at 37°C for 24 hours.
1 mL of a supernatant was collected by a low-adsorptive tube after mixing with inversion. The collected solution was filtered through a 0.22 $\mu$m filter to obtain a measurement sample.
Gel permeation chromatography was carried out on 5 $\mu$L of the measurement sample under the following conditions to obtain an elution curve in which the vertical axis is the absorbance and the horizontal axis is the elution time.
Gel permeation chromatography was also performed for substances having a known molecular weight (CBE3 : 51.6 kDa, GPC standard product: 43 kDa, deoxyribonuclease: 28 kDa, lysozyme: 14 kDa) under the same conditions, and the relationship between the elution time and the molecular weight was grasped.
The elution curves obtained from the tissue repair materials of Examples 1 to 5 are shown in Figs. 1 to 5, and the elution curves obtained from the tissue repair material of Comparative Example 1 are shown in Fig. 6.

(Conditions)

Column: ACQUITY UPLC Protein BEH SEC Column, 200 Å, 1.7 $\mu$m, 4.6 mm $\times$ 150 mm
Column temperature: 30°C
Column length: 150 mm
Column inner diameter: 4.6 mm
Filler: ethylene bridged (BEH) particles
Particle size of the filler: 1.7 $\mu$m
Pore size of the filler: 200 Å
Mobile phase: 0.1 mol/L sodium sulfate, 0.01 mol/L sodium dihydrogen phosphate, and 1% by mass of sodium dodecyl sulfate, pH 5.3
Flow rate: 0.1 mL/minute
Detector: wavelength-tunable UV detector (TUV)
Measuring wavelength: 215 nm
Adjustment of measurement sample: The tissue repair material is filled into a tube having a capacity of 15 mL, 5 mL of water for injection is added to the tube, and the tube is incubated at 37°C for 24 hours. 1 mL of a supernatant is collected by a low-adsorptive tube after mixing with inversion and filtered through a 0.22 $\mu$m filter.
Amount of the measurement sample: 5 $\mu$L

In the elution curve obtained from the tissue repair material of Examples 1 to 7, an absorption peak (referred to as the number of absorption peaks in Table 1) at an absorbance of 0.025 or more was observed in at least two of a molecular weight range of 43 kDa or more and 51 kDa or less, a molecular weight range of 28 kDa or more and less than 43 kDa, and a molecular weight range of 14 kDa or more and less than 28 kDa.

In the elution curve of the tissue repair material of Comparative Examples 1 and 2, no absorption peak with an absorbance of 0.025 or more was observed in any of the molecular weight range of 43 kDa or more and 51 kDa or less, the molecular weight range of 28 kDa or more and less than 43 kDa, and the molecular weight range of 14 kDa or more and less than 28 kDa.

From the elution curves of the tissue repair materials of Examples and Comparative Examples obtained as described above, a ratio of an area at a molecular weight of 14 kDa or more and 43 kDa or less to an area at a molecular weight of more than 43 kDa and 51 kDa or less was obtained and summarized in Table 1 (in Table 1, the ratio is described as an area ratio).

<<Measurement of water absorption rate of tissue repair material>>

First, a filter cup in which the mass (w0) had been measured was set in a microtube (hereinafter, referred to as a tube).

Next, 500 μL of water was added to the filter cup, and stirred for 2 hours by a rotator.

After stirring, the tube was centrifuged under the conditions of 25°C and 6,000 × g for 1 minute, and it was confirmed that water had moved from the filter cup to the inside of the tube. The mass (w1) of the filter cup was measured again, and the residual water amount was calculated using the following expression.

Residual water amount (mg): w1 - w0

Next, another filter cup in which the mass (w2) had been measured was set in the tube, and 10 mg (mass: w3) of the tissue repair material was filled in a filter cup.

Next, 500 μL of water was added to the filter cup, and stirred for 2 hours by a rotator.

After stirring, the tube was centrifuged under the conditions of 25°C and 6,000 × g for 1 minute, and it was confirmed that water had moved from the filter cup to the inside of the tube.

The sum (w4) of the mass of the tissue repair material remaining in the filter cup and the mass of the filter cup was measured, and the water absorption rate was calculated using Expression (1). The results are summarized in Table 1.

$$\text{Water absorption rate (\%)} = (w4 - w2 - (w1 - w0))/w3 \times 100 \ .... \ (1)$$

<<Measurement of acidolysis residual rate of tissue repair material>>

A microtube (hereinafter, referred to as a tube) was prepared, and a mass (A) thereof was measured.

15.0 (±0.2) mg of the tissue repair material produced in Examples and Comparative Examples was weighed (mass: B), and filled in a tube.

1.7 mL of 1 mol/L hydrochloric acid was added to the tube containing the tissue repair material, and the tube was heated for 3 hours using a heat block set at 37°C.

After the heating, the tube was placed on ice to stop the acidolysis reaction, and centrifuged for 1 minute under conditions of 10,000 × g using a centrifuge set to 4°C in advance.

In the tube, it was confirmed that the tissue repair material was precipitated, the supernatant was suctioned, 1 mL of ultrapure water, which had been cooled on ice in advance, was added thereto, and the mixture was centrifuged again under the same conditions as described above.

After the centrifugation, the supernatant was suctioned, ultrapure water was added to the tube again, and the tube was centrifuged again under the same conditions as described above.

After the centrifugation, the supernatant was suctioned, and the tube filled with the tissue repair agent was allowed to stand in a -80°C freezer (manufactured by Nihon Freezer Co., Ltd., ultra-low temperature freezer CLN-31UW) for 1 hour or more to obtain a frozen tissue repair agent. The tissue repair agent was transferred to a vacuum freeze dryer (manufactured by TOKYO RIKAKIKAI CO., LTD., FDU-1110), and vacuum freeze-dried for 16 hours to 24 hours in a state where the vacuum degree was lowered to about 10 Pa.

After the freeze-drying, the tube was taken out from the freeze-dryer, and the cap of the tube was quickly closed in order to prevent the tissue repair material from absorbing moisture in the air.

The mass (C) of the tube was measured, and the acidolysis residual rate was calculated using Expression (2). The results are summarized in Table 1.

$$\text{Acidolysis residual rate (\%)} = (C - A)/B \times 100 \ .... \ (2)$$

<<Evaluation of tissue repair ability>>

A circular bone defect portion having a diameter of 5 mm was created in the parietal bone of the SD rat, 3.0 ± 0.2 mg of the tissue repair material manufactured in Examples and Comparative Examples was filled in the bone defect portion, and the tissue repair material was sutured together with the periosteum.

After 2 weeks and 4 weeks after the surgery, the bone mass of the rat parietal bone was measured by micro-CT, and a ratio of the bone volume inside the defect portion to the defect portion volume was obtained. The results are summarized in Table 1.

[Table 1]

| | Number of absorption peaks | Area ratio | Water absorption rate (%) | Acidolysis residual rate (%) | Evaluation of tissue repair ability | |
|---|---|---|---|---|---|---|
| | | | | | After two weeks | After four weeks |
| Example 1 | 4 | 3.00 | 691 | 21 | 29.3 | 78.6 |
| Example 2 | 4 | 3.22 | 819 | 7 | 50.0 | 80.5 |
| Example 3 | 1 | 8.81 | 529 | 56 | 33.7 | 79.2 |
| Example 4 | 2 | 7.52 | 699 | 49 | 43.3 | 77.2 |
| Example 5 | 1 | 9.16 | 559 | 51 | 31.5 | 85.4 |
| Comparative Example 1 | 0 | 2.06 | 538 | 40 | 29.2 | 70.0 |

From Table 1, it can be seen that the tissue repair material obtained in Examples had excellent tissue repairability as compared with the tissue repair material obtained in Comparative Examples. In addition, from Table 1, it can be seen that the tissue repair material obtained in Examples also had excellent tissue repair speed.

The disclosures of Japanese Patent Application No. 2022-057374 filed on March 30, 2022 and Japanese Patent Application No. 2022-062058 filed on April 1, 2022 are incorporated herein by reference in their entirety. All documents, patent applications, and technical standards disclosed in this specification are incorporated in this specification by reference such that the incorporation of each of the documents, the patent applications, and the technical standards by reference is specific and is as detailed as each of the documents, the patent applications, and the technical standards. [Sequence list] International application 21F01743W1JP22039789_1.xml based on International Patent Cooperation Treaty

**Claims**

1. A tissue repair material comprising:

   crosslinked gelatin,
   wherein in an elution curve of the crosslinked gelatin, which is obtained by gel permeation chromatography under the following conditions, at least one absorption peak with an absorbance of 0.025 or more is present in a molecular weight range of 14 kDa or more and 51 kDa or less,
   (conditions)
   column length: 150 mm
   column inner diameter: 4.6 mm
   filler: ethylene bridged (BEH) particles
   particle size of the filler: 1.7 $\mu$m
   pore size of the filler: 200 Å
   column temperature: 30°C
   mobile phase: 0.1 mol/L sodium sulfate, 0.01 mol/L sodium dihydrogen phosphate, and 1% by mass of sodium dodecyl sulfate, pH 5.3
   flow rate: 0.1 mL/minute
   detector: wavelength-tunable UV detector (TUV)
   measuring wavelength: 215 nm
   adjustment of measurement sample: the tissue repair material is charged into a tube having a capacity of 15 mL, 5 mL of water for injection is added to the tube, and the tube is incubated at 37°C for 24 hours, and 1 mL of a supernatant is collected by a low-adsorptive tube after mixing with inversion and filtered through a 0.22 μm filter amount of the measurement sample: 5 μL.

2. The tissue repair material according to claim 1,
wherein an absorption peak is present in a molecular weight range of 14 kDa or more and less than 28 kDa and an absorption peak is present in a molecular weight range of 28 kDa or more and less than 43 kDa.

3. The tissue repair material according to claim 1,
wherein a water absorption rate of the crosslinked gelatin is 450% or more.

4. The tissue repair material according to claim 1,
wherein a residual rate of the crosslinked gelatin after a decomposition treatment for 3 hours using 1 mol/liter (L) hydrochloric acid is 60% or less.

5. The tissue repair material according to claim 1,
wherein the gelatin is one or more selected from the group consisting of a peptide consisting of an amino acid sequence set forth in SEQ ID NO: 1, a peptide consisting of an amino acid sequence in which one or several amino acids are deleted, substituted, or added from the amino acid sequence set forth in SEQ ID NO: 1, and having bioaffinity, and a peptide consisting of an amino acid sequence having amino acid sequence identity of 80% or more with a partial amino acid sequence consisting of the 4th to 192nd amino acid residues in the amino acid sequence set forth in SEQ ID NO: 1, and having bioaffinity.

6. The tissue repair material according to claim 1,
wherein in the elution curve of the crosslinked gelatin, a ratio of an area at a molecular weight of 14 kDa or more and 43 kDa or less to an area at a molecular weight of more than 43 kDa and 51 kDa or less is 2.5 or more.

7. A manufacturing method of a tissue repair material comprising:

a step of heating gelatin under conditions of 100°C to 170°C and 2 hours to 24 hours to obtain crosslinked gelatin; and
a step of irradiating the crosslinked gelatin with radiation having a dose of 5 kGy to 50 kGy.

8. The manufacturing method of a tissue repair material according to claim 7,
wherein the radiation is a γ-ray or an electron beam.

9. The manufacturing method of a tissue repair material according to claim 7,

wherein, in a molecular weight range of 14 kDa or more and 51 kDa or less in an elution curve of the crosslinked gelatin in a tissue repair material manufactured by the manufacturing method of a tissue repair material according to claim 7, which is obtained by gel permeation chromatography under the following conditions, an absorption peak is present in a molecular weight range of 14 kDa or more and less than 28 kDa and an absorption peak is present in a molecular weight range of 28 kDa or more and less than 43 kDa,
(conditions)
column length: 150 mm
column inner diameter: 4.6 mm
filler: ethylene bridged (BEH) particles
particle size of the filler: 1.7 μm
pore size of the filler: 200 Å
column temperature: 30°C
mobile phase: 0.1 mol/L sodium sulfate, 0.01 mol/L sodium dihydrogen phosphate, and 1% by mass of sodium dodecyl sulfate, pH 5.3
flow rate: 0.1 mL/minute
detector: wavelength-tunable UV detector (TUV)
measuring wavelength: 215 nm
adjustment of measurement sample: the tissue repair material is charged into a tube having a capacity of 15 mL, 5 mL of water for injection is added to the tube, and the tube is incubated at 37°C for 24 hours, and 1 mL of a supernatant is collected by a low-adsorptive tube after mixing with inversion and filtered through a 0.22 μm filter amount of the measurement sample: 5 μL.

**10.** The manufacturing method of a tissue repair material according to claim 7,
wherein a water absorption rate of the crosslinked gelatin after the irradiation with radiation is 450% or more.

**11.** The manufacturing method of a tissue repair material according to claim 7,
wherein an acidolysis residual rate of the crosslinked gelatin after the irradiation with radiation is 60% or less.

**12.** The manufacturing method of a tissue repair material according to claim 7,

wherein the crosslinked gelatin after the irradiation with radiation is a porous body, and
a void ratio of the crosslinked gelatin after the irradiation with radiation is 80.00% to 99.99%.

**13.** The manufacturing method of a tissue repair material according to claim 7,
wherein the gelatin is one or more selected from the group consisting of a peptide consisting of an amino acid sequence set forth in SEQ ID NO: 1, a peptide consisting of an amino acid sequence in which one or several amino acids are deleted, substituted, or added from the amino acid sequence set forth in SEQ ID NO: 1, and having bioaffinity, and a peptide consisting of an amino acid sequence having amino acid sequence identity of 80% or more with a partial amino acid sequence consisting of the 4th to 192nd amino acid residues in the amino acid sequence set forth in SEQ ID NO: 1, and having bioaffinity.

# FIG. 1

# FIG. 2

# FIG. 3

# FIG. 4

# FIG. 5

# FIG. 6

<table>
<tr><td colspan="2" align="center">**INTERNATIONAL SEARCH REPORT**</td><td>International application No.<br><br>**PCT/JP2022/039789**</td></tr>
</table>

**A.     CLASSIFICATION OF SUBJECT MATTER**

*A61L 27/22*(2006.01)i; *A61L 27/56*(2006.01)i; *C07K 14/78*(2006.01)n; *C12N 15/12*(2006.01)n
FI:     A61L27/22 ZNA; A61L27/56; C07K14/78; C12N15/12

According to International Patent Classification (IPC) or to both national classification and IPC

**B.     FIELDS SEARCHED**

Minimum documentation searched (classification system followed by classification symbols)

A61L27/22; A61L27/56; C07K14/78; C12N15/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.     DOCUMENTS CONSIDERED TO BE RELEVANT**

| Category* | Citation of document, with indication, where appropriate, of the relevant passages | Relevant to claim No. |
|---|---|---|
| X | JP 2014-124416 A (GUNZE LTD.) 07 July 2014 (2014-07-07) experimental example 1 | 1-4, 6-12 |
| Y | experimental example 1 | 5, 13 |
| Y | WO 2013/137268 A1 (FUJIFILM CORP.) 19 September 2013 (2013-09-19) example 1 | 5, 13 |

☐ Further documents are listed in the continuation of Box C.        ☑ See patent family annex.

| | |
|---|---|
| *     Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **14 November 2022** | **29 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/039789**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "The form of Annex C/ST.25 text file" above shall read as "the form of ST.26.".

Form PCT/ISA/210 (continuation of first sheet) (January 2015)

**INTERNATIONAL SEARCH REPORT**
Information on patent family members

International application No.

**PCT/JP2022/039789**

| Patent document cited in search report | | | Publication date (day/month/year) | Patent family member(s) | | | Publication date (day/month/year) |
|---|---|---|---|---|---|---|---|
| JP | 2014-124416 | A | 07 July 2014 | (Family: none) | | | |
| WO | 2013/137268 | A1 | 19 September 2013 | US | 2014/0378662 | A1 | |
| | | | | example 1 | | | |
| | | | | EP | 2826494 | A1 | |
| | | | | KR | 10-2014-0122761 | A | |
| | | | | CN | 104244999 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013137268 A
- EP 1014176 A2
- US 6992172 B1
- WO 200485473 A2
- WO 2008103041 A1
- JP 2010519293 A
- JP 2010519252 A
- JP 2010518833 A
- JP 2010519251 A
- WO 2010128672 A1
- WO 2010147109 A1
- JP 2022057374 A
- JP 2022062058 A
- JP 22039789 B